Europäisches Patentamt

European Patent Office

Office européen des brevets

(11) Veröffentlichungsnummer: **0 484 786 A1**

# EUROPÄISCHE PATENTANMELDUNG

(21) Anmeldenummer: **91118348.1**

(51) Int. Cl.5: **C07D 323/06**

(22) Anmeldetag: **28.10.91**

(30) Priorität: **08.11.90 DE 4035495**

(43) Veröffentlichungstag der Anmeldung:
**13.05.92 Patentblatt 92/20**

(84) Benannte Vertragsstaaten:
**AT BE CH DE DK ES FR GB GR IT LI LU NL SE**

(71) Anmelder: **HOECHST AKTIENGESELLSCHAFT**
**Postfach 80 03 20**
**W-6230 Frankfurt am Main 80(DE)**

(72) Erfinder: **Mück, Karl-Friedrich, Dr.**
**Schnitterweg 7**
**W-6200 Wiesbaden(DE)**
Erfinder: **Reuschel, Gerhard, Dr.**
**Am Flachsland 56**
**W-6233 Kelkheim (Taunus)(DE)**
Erfinder: **Fleischer, Dietrich, Dr.**
**Seitersweg 19**
**W-6100 Darmstadt(DE)**

(54) **Verfahren zur Herstellung von Trioxan.**

(57) Trioxan kann mit minimalem Energiebedarf gewonnen werden, wenn Acetalpolymere in Gegenwart von Wasser und einem sauren Katalysator abgebaut werden. Das Verfahren stellt einen Beitrag zur Abfallbeseitigung und zum Umweltschutz dar.

EP 0 484 786 A1

Die Erfindung betrifft ein Verfahren zur Herstellung von Trioxan aus Homo- und/oder Copolymeren des Formaldehyds in Gegenwart saurer Katalysatoren.

Die Herstellung von Trioxan aus wäßrigen Formaldehydlösungen ist verschiedentlich in der Literatur beschrieben (vgl. Walker, Formaldehyde, Reinhold Publ. New York, 3. Auflage, 1964, Seite 198-199).

Trioxan (TOX) wird bei höheren Temperaturen in Gegenwart saurer Katalysatoren aus wäßrigem Formaldehyd gebildet und durch Destillation aus dem Reaktionsgemisch entfernt. Die Aufarbeitung des Synthesedampfs wird meist in einer dem Reaktor aufgesetzten Verstärkerkolonne durchgeführt (US-PS 2,304,080). Die trioxanreiche Phase wird einer Extraktion und/oder einem anderen Trennverfahren unterworfen. Um hohe Raum-Zeit-Ausbeuten zu erreichen, wird die Reaktion in einem Zwangsumlaufverdampfer durchgeführt (DE-PS 28 53 091). Die durch Trioxancopolymerisation erhaltenen Copolymerisate haben ein hohes Qualitätsniveau und gehören in das Gebiet der technischen Kunststoffe.

Technische Kunststoffe werden durch Extrusion oder Spritzguß verarbeitet, wobei Produktabfälle entstehen, die entsorgt werden müssen. Bei der Spritzgußverarbeitung fallen Angüsse und Grate an. Extrusionsware wird zum Großteil spanend bearbeitet, wobei nicht selten bis zu 50 % des Materials als Abfall erhalten werden. Auch während des Produktionsablaufs fallen mitunter Materialien an, die nicht immer typgerecht sind und entsorgt werden müssen. Als Entsorgung bietet sich bisher die Verbrennung und das Deponieren an. Ein Recycling-Verfahren ist daher vorzuziehen.

Ein Verfahren zur Erzeugung von gasförmigem Formaldehyd aus Polyoxymethylen, wie Paraformaldehyd, ist bekannt (DE-PS 22 39 266). Hierbei wird eine Dispersion aus polymerem Formaldehyd bei 100 - 300°C thermisch zu Formaldehyd zersetzt und dieser gasförmig abgetrennt. Dieses Verfahren ist äußerst kompliziert und bedarf eines hohen technischen Aufwands, um Verstopfungen durch Paraformaldehyd zu verhindern. Beim Einsatz von Copolymerisaten ist eine Verunreinigung des Formaldehyds durch Comonomere nicht auszuschließen.

Aufgabe der vorliegenden Erfindung war es, ein Verfahren zu finden, das u.a. ein Recycling von Polyacetal ermöglicht, ohne die genannten Schwierigkeiten aufzuweisen, und ein Material zu erzeugen, das direkt zur Copolymerisation wieder eingesetzt werden kann.

Dies gelang durch ein Verfahren zur kontinuierlichen Herstellung von Trioxan aus Formaldehyd, bei dem ein Acetalpolymer in Gegenwart von Wasser und einem sauren Katalysator abgebaut und der entstandene Formaldehyd im gleichen Verfahrensschritt in Trioxan überführt wird.

Nach dem erfindungsgemäßen Verfahren werden hohe Trioxankonzentrationen im Synthesedampf erhalten, die denen des maximalen Gleichgewichtswertes entsprechen, der beim Einsatz von Formaldehydlösungen erzielt werden kann.

Die Trioxanherstellung gemäß der Erfindung erfolgt durch Umsatz von Homo- und/oder Copolymerisaten des Formaldehyds und gegebenenfalls cyclischer Formale, z.B. in Form eines Recyclisats in Gegenwart von Wasser und saurem Katalysator. Die Wasserzugabe wird so bemessen, daß sich m Reaktor rechnerisch ein Formaldehydgehalt von 50 - 90, vorzugsweise 65 - 85 Gew.-% einstellt. Ein Zusatz von Antischaummittel kann hilfreich sein. Als Katalysator werden Mineralsäuren, starke organische Säuren oder eine in ihrer katalytischen Aktivität entsprechende Menge eines anderen sauren Katalysators verwendet. Als saure Katalysatoren, die im allgemeinen weniger flüchtig als das Reaktionsgemisch sein müssen, haben sich besonders Schwefelsäure, Phosphorsäure, p-Toluolsulfonsäure oder stark saure Ionenaustauscher, z.B. Polystyrolaustauscher mit Sulfonsäuregruppen, als brauchbar erwiesen. Die Katalysatormenge ist nicht kritisch und beträgt in der Regel 1 bis 60, vorzugsweise 10 - 50 Gew.-%, bezogen auf die Reaktionsmischung.

Als Recyclisat werden Homo- und Copolymerisate des Formaldehyds und/oder cyclischer Formale, vorzugsweise Copolymerisate, in zerkleinerter Form eingesetzt. Die Recyclisate können auch Farbstoffe, Pigmente, Stabilisatoren und andere übliche Zusätze enthalten. Das Recyclisat wird z.B. über eine Feststoff-Schleuse separat oder zusammen mit Wasser in den Reaktor eindosiert. Die Zusätze, z.B. Stabilisatoren stören die Umsetzung nicht. Im Reaktor bilden sich auch die Comonomere zurück, die gemeinsam mit dem Trioxan aufgearbeitet werden. Die Auftrennung der Comonomeren ist Stand der Technik. Die Reaktion wird erfindungsgemäß in einem bekannten Umlaufreaktor mit Verdampfer durchgeführt. Hierfür eignen sich beispielsweise Zwangsumlaufverdampfer, Fallfilmverdampfer, Rising-Filmverdampfer oder Dünnschichtverdampfer mit Zwangsumlauf. Derartige Systeme sind zum Beispiel in Ullmann, Band 1 (1951), 3. Auflage, Seite 533 - 537, beschrieben. Besonders geeignet sind Zwangsumlaufverdampfer. Diese Reaktoren müssen aber Einbauten enthalten, um ein Ansaugen der Feststoffpartikel durch die Pumpe des Zwangsumlaufreaktors zu vermeiden. Geeignet sind hierzu korbähnliche Einbauten oder Siebe vor den Pumpen.

Die Verweilzeit der Reaktionsmischung im Reaktionssystem beträgt 5 - 240 Minuten, vorzugsweise 15 - 60 Minuten. Die Temperatur der Reaktionsmischung beträgt je nach Druck 50 bis 150°C, vorzugsweise 95 bis 130°C.

Das Reaktionsgemisch, bestehend aus Trioxan, Formaldehyd und Wasser, sowie gegebenenfalls cyclischen Formalen, wird mit Hilfe des Verdampfers destillativ aus dem Reaktionssystem entfernt. Hierbei kann bei Normaldruck, unter vermindertem Druck, beispielsweise bei 300 bis 1000 mbar, oder unter Überdruck, beispielsweise 1 bis 4 bar, gearbeitet werden. Vorzugsweise wird bei 1 bis 2 bar gearbeitet.

Der das Reaktionssystem verlassende Synthesedampf wird in üblicher Weise entweder als Dampf oder als Kondensat mittels einer Rektifikation, wie in der GB-PS 1 012 372 beschrieben, angereichert.

Die anfallende trioxanreiche Fraktion, die gegebenenfalls auch cyclische Formale enthält, kann dann z.B. durch Extraktion mit einem mit Wasser nicht mischbaren Lösemittel für Trioxan (und gegebenenfalls für die cyclischen Formale) wie Methylenchlorid und anschließende Neutralisation und fraktionierte Destillation oder Kristallisation gereinigt werden. Auch andere bekannte Trennverfahren können hierfür Einsatz finden (Process Economics Program Stanford Institute Report 23 (1967) 181 oder DE-OS 15 70 335)). Die vom Trioxan befreiten Produktströme, die hauptsächlich noch Formaldehyd und Wasser enthalten, können kontinuierlich in den Reaktionsbehälter zurückgeführt werden.

Das Verfahren gemäß der Erfindung,das kontinuierlich oder diskontinuierlich durchgeführt werden kann, ermöglicht eine Trioxansynthese mit minimalem Energiebedarf, da die Herstellkosten für die Bereitstellung von hochkonzentriertem Formaldehyd entfallen. Der hochkonzentrierte Formaldehyd wird hierbei durch das eingesetzte Recyclisat ersetzt. Das Verfahren stellt einen Beitrag zur Abfallbeseitigung und zum Umweltschutz dar, da hierdurch Abfallstoffe dem Produktionsprozeß zugeführt und damit Verbrennungsanlagen und Deponien entlastet werden, über die bisher das Recyclisat entsorgt werden mußte.

Beispiele:

In einem 2-l-Vierhalskolben mit Rührer wurden 250 g Wasser, 250 g konzentrierte Schwefelsäure und 500 g Recyclisat in Granulatform (Copolymerisat aus Trioxan und 3 Gew.-% Dioxolan) vorgelegt. Die Mischung wurde zum Sieden erhitzt und das Destillat in einem Quenchkühler kondensiert. In Abständen von jeweils 15 Minuten wurden weitere 39 g Granulat und 21 g Wasser (entsprechend einer 65 %igen Formaldehydlösung) getrennt in den Kolben gebracht. Die gesamte Reaktionsdauer betrug 5 Stunden. Die Destillatzusammensetzung wurde stündlich analysiert. Die Mittelwerte der Versuche sind in der Tabelle angegeben. Der Dioxolan-Gehalt entspricht dem Comonomeren-Gehalt im eingesetzten Granulat. Der Trioxangehalt in den Beispielen gemäß der Erfindung entspricht demjenigen, wie er nach dem Stand der Technik im herkömmlichen Trioxan-Verfahren aus wäßrigem Formaldehyd erhalten wird (Vergleichsbeispiel).

EP 0 484 786 A1

Tabelle

Bedingungen: Vorgelegt im Kolben: 250 g konz. Schwefelsäure;
250 g Wasser, 500 g Granulat
Temperatur: 104°C, Laufzeit 5 h
Zuspeisung alle 1/4 h: 39 g Granulat, 21 g Wasser

| Beispiel | Durchsatz g/h | TOX im Dest. % | $CH_2O$ im Dest. % | Dioxolan im Dest. % | Wasser als Diff.[**] % |
|---|---|---|---|---|---|
| 1 | 201 | 25,7 | 39,8 | 3,1 | 31,4 |
| 2 | 223 | 19,7 | 40,1 | 2,4 | 37,8 |
| Vergleich[*] | 250 | 20,6 | 41,9 | --- | 37,5 |

[*]  Formaldehydzuspeisekonzentration 63,5 %, Schwefelsäuregehalt im Reaktorsumpf 10 %
[**] zu 100 %

**Patentansprüche**

1. Verfahren zur Herstellung von Trioxan aus Formaldehyd, dadurch gekennzeichnet, daß ein Acetalpolymer in Gegenwart von Wasser und einem sauren Katalysator abgebaut und der entstandene Formaldehyd im gleichen Verfahrensschritt in Trioxan überführt wird.

2. Verfahren nach Anspruch 1, dadurch gekennzeichnet, daß als Acetalpolymer ein Homo- und/oder
Copolymerisat des Formaldehyds und gegebenenfalls cyclischer Formale eingesetzt wird.

3. Verfahren nach Anspruch 1 oder 2, dadurch gekennzeichnet, daß eine Mischung aus Trioxan und
mindestens einem cyclischen Formal erhalten wird.

4. Verfahren nach einem oder mehreren der Ansprüche 1 bis 3, dadurch gekennzeichnet, daß ein
Recyclisat eingesetzt wird.

5. Verfahren nach einem oder mehreren der Ansprüche 1 bis 4, dadurch gekennzeichnet, daß das
Acetalpolymer ein Trioxancopolymer mit Dioxolan/Ethylenoxid oder mit Butandiolformal als Comonomerbestandteilen ist.

6. Verfahren nach einem oder mehreren der Ansprüche 1 bis 4, dadurch gekennzeichnet, daß es
kontinuierlich durchgeführt wird.

7. Verfahren nach einem oder mehreren der Ansprüche 1 bis 6, dadurch gekennzeichnet, daß die
Umsetzung in einem Zwangsumlaufreaktor durchgeführt wird.

8. Verfahren nach einem oder mehreren der Ansprüche 1 bis 7, dadurch gekennzeichnet, daß die
Reaktionstemperatur 50 bis 150, vorzugsweise 95 bis 130°C beträgt.

9. Verfahren nach einem oder mehreren der Ansprüche 1 bis 8, dadurch gekennzeichnet, daß die
Wassermenge so groß ist, daß der Formaldehydgehalt im Reaktor rechnerisch 50 - 90, vorzugsweise
65 bis 85 Gew.-% beträgt.

4

10. Verfahren nach einem oder mehreren der Ansprüche 1 bis 9, dadurch gekennzeichnet, daß als saurer Katalysator Schwefelsäure, Phosphorsäure, p-Toluolsulfonsäure oder ein saurer Ionenaustauscher in Mengen von 1 bis 60, vorzugsweise 10 bis 50 Gew.-%, bezogen auf die Reaktionsmischung,eingesetzt wird.

11. Verfahren nach Anspruch 10, dadurch gekennzeichnet, daß als Katalysator konz. Schwefelsäure eingesetzt wird.

12. Verfahren nach einem oder mehreren der Ansprüche 1 bis 11, dadurch gekennzeichnet, daß die Reaktionsprodukte durch Destillation bei einem Druck von 1 bis 2 bar abgetrennt werden.

Europäisches
Patentamt

**EUROPÄISCHER RECHERCHENBERICHT**

Nummer der Anmeldung

EP    91 11 8348

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | DE-A-2 064 100 (BASF)<br><br>* Seite 1 - Seite 2; Beispiel 1 *<br><br>----- | 1,4,8,<br>10-12 | C07D323/06 |
| | | | **RECHERCHIERTE SACHGEBIETE (Int. Cl.5)**<br><br>C07D |

Der vorliegende Recherchenbericht wurde für alle Patentansprüche erstellt

| Recherchenort | Abschlußdatum der Recherche | Prüfer |
|---|---|---|
| DEN HAAG | 21 JANUAR 1992 | RUSSELL F. ENGLISH |

KATEGORIE DER GENANNTEN DOKUMENTE

X : von besonderer Bedeutung allein betrachtet
Y : von besonderer Bedeutung in Verbindung mit einer
     anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
     nach dem Anmeldedatum veröffentlicht worden ist
D : in der Anmeldung angeführtes Dokument
L : aus andern Gründen angeführtes Dokument
.........................................................................
& : Mitglied der gleichen Patentfamilie, übereinstimmendes
     Dokument

EPO FORM 1503 03.82 (P0403)